# EUROPÄISCHE PATENTSCHRIFT

(11) **EP 2 478 117 B1**
(45) Veröffentlichungstag und Bekanntmachung des Hinweises auf die Patenterteilung: **31.07.2013**
(21) Anmeldenummer: 10765749.6
(22) Anmeldetag: 17.09.2010
(51) Int. Cl.: C12Q 1/68

(54) **Verfahren zur Früherkennung von Zervixkarzinomen**
Method for early diagnosis of cervical carcinomas
Procédé de diagnostic précoce de carcinomes cervicaux

(30) Priorität: 18.09.2009 EP 09011941
(43) Veröffentlichungstag der Anmeldung: 25.07.2012
(73) Patentinhaber: oncgnostics GmbH, 07745 Jena (DE)
(72) Erfinder: DÜRST, Matthias, 07743 Jena (DE); HANSEL, Alfred, 07745 Jena (DE); STEINBACH, Daniel, 08115 Lichtentanne (DE)
(74) Vertreter: Schüssler, Andrea
(86) Internationale Anmeldenummer: PCT/EP2010/005735
(87) Internationale Veröffentlichungsnummer: WO 2011/032721

(56) Entgegenhaltungen:
- US-A1- 2007 026 393
- WANG SOPHIA S ET AL: "Identification of novel methylation markers in cervical cancer using restriction landmark genomic scanning.", CANCER RESEARCH 1 APR 2008, Bd. 68, Nr. 7, 1. April 2008 (2008-04-01), Seiten 2489-2497, XP002566925, ISSN: 1538-7445
- HUANG YI-WEN ET AL: "Hypermethylation of CIDEA and RXFP3 as potential epigenetic markers for endometrial cancer", PROCEEDINGS OF THE AMERICAN ASSOCIATION FOR CANCER RESEARCH ANNUAL MEETING, Bd. 49, April 2008 (2008-04), Seite 12, XP009128984, & 99TH ANNUAL MEETING OF THE AMERICAN-ASSOCIATION-FOR-CANCER-RESEARCH; SAN DIEGO, CA, USA; APRIL 12 -16, 2008 ISSN: 0197-016X
- AUSCH C ET AL: "Comparative analysis of PCR-based biomarker assay methods for colorectal polyp detection from fecal DNA", CLINICAL CHEMISTRY 20090801 AMERICAN ASSOCIATION FOR CLINICAL CHEMISTRY INC. USA LNKD- DOI:10.1373/CLINCHEM.2008.122937, Bd. 55, Nr. 8, 18. Juni 2009 (2009-06-18), Seiten 1559-1563, XP002610021, ISSN: 0009-9147
- JACOBS M V ET AL: "Group-specific differentiation between high and low-risk human papillomavirus genotypes by general primer-mediated PCR and two cocktails of oligonucleotide probes", JOURNAL OF CLINICAL MICROBIOLOGY, AMERICAN SOCIETY FOR MICROBIOLOGY, WASHINGTON, DC, US, Bd. 33, Nr. 4, 1. April 1995 (1995-04-01), Seiten 901-905, XP002241733, ISSN: 0095-1137
- MUELLER HANNES M ET AL: "A DNA methylation pattern similar to normal tissue is associated with better prognosis in human cervical cancer", CANCER LETTERS, Bd. 209, Nr. 2, 25. Juni 2004 (2004-06-25) , Seiten 231-236, XP002566926, ISSN: 0304-3835
- ESTELLER MANEL: "Epigenetic gene silencing in cancer: the DNA hypermethylome", HUMAN MOLECULAR GENETICS, Bd. 16, Nr. Sp. Iss. 1, April 2007 (2007-04), Seiten R50-R59, XP002566927, in der Anmeldung erwähnt

## Beschreibung

Die vorliegende Erfindung betrifft ein Verfahren zur frühen Diagnose von Karzinomen des Zervixkarzinoms, sowie deren Vorstufen. Das Verfahren basiert auf der Bestimmung des Methylierungsstatus von Abschnittten der Genregionen von ASTN1 (Astrotactin 1) und ZNF671 (Zinkfinger-Protein 671, Transkriptionsfaktor).Eine DNA-Methylierung in Guanin/Cytosin-reichen Bereichen, den sogenannten CpG-Inseln, im Promotorbereich und/oder der 5'-Region wenigstens eines dieser Gene bzw. beider Gene ist charakteristisch für Karzinome oder Karzinomvorstufen des Zervixkarzinoms. Der Nachweis entsprechend veränderter DNA wird diagnostisch genutzt. Die vorliegende Erfindung betrifft außerdem Kits, die die Durchführung des erfindungsgemäßen Diagnoseverfahrens gestatten.

Gebärmutterhalskrebs (Zervixkarzinom) ist die zweithäufigste bösartige Krebserkrankung bei Frauen weltweit. Er entwickelt sich im Zuge einer Infektion mit sog. Hochrisiko-humanen-Papillomaviren (hr-HPV) über Vorstufen, die als zervikale intraepitheliale Neoplasien (CIN) bezeichnet werden. CIN1: (leichte Dysplasie) geht von basal aus bis höchstens einem Drittel der Höhe des Epithels; CIN2: (mittelgradige Dysplasie) bis zu zwei Drittel des Epithels; CIN3: (hochgradige Dysplasie) durchzieht fast das gesamte Epithel. CIN2 und CIN3 werden als schwergradige Dysplasien bezeichnet. Letztere haben ein hohes Risiko in ein Zervixkarzinom überzugehen. In der vorliegenden Anmeldung werden CIN2, CIN3 und Zervixkarzinom unter dem Begriff CIN2+ zusammengefasst. Neben einer hr-HPV Infektion sind auch weitere Faktoren an der Zervixkarzinogenese beteiligt.

Der bestehende Vorsorgetest für den Nachweis eines Zervixkarzinoms und dessen Vorstufen (CIN) beruht auf einem zytomorphologischen Verfahren (Pap-Test). Der Pap-Test ist höchst anfällig für Fehler, da wenige Krebs- oder CINverdächtige Zellen in einem Hintergrund tausender normaler Zellen mikroskopisch erkannt werden müssen. Des Weiteren ist die Beurteilung der Zellmorphologie äußerst subjektiv. Diese Schwächen führen dazu, dass die Sensitivität des Pap-Tests für den Nachweis von CIN2+ 53% und die Spezifität 96,3% beträgt (Cuzick et al., 2006; Int J Cancer, 119:1095-1101).

Durch molekulare Testverfahren konnte die Krebsvorsorge wesentlich verbessert werden. Da bis auf wenige Ausnahmen alle Zervixkarzinome und deren Vorstufen hr-HPV-DNA enthalten, erscheint HPV-DNA als idealer Marker für die Krebsvorsorge. In verschiedenen veröffentlichten Studien konnte gezeigt werden, dass der HPV-DNA-Test (ein PCR-Verfahren) für den Nachweis von CIN2+ eine Sensitivität von 96,1% und eine Spezifität von 90,7% aufweist. Allerdings ist der positive Vorhersagewert eines HPV-DNA-Tests für CIN2+ mit nur 20,3% erwartungsgemäß schlecht, da viele Frauen lediglich mit HPV infiziert sind, aber keine Krebsvorstufen aufweisen (Cuzick et al., 2006; Int J Cancer, 119:1095-1101). Somit besteht ein Bedarf an einer verbesserten Diagnostik von Karzinomen des Anogenitaltraktes, insbesondere des Zervixkarzinoms.

Wang et al., Cancer Res. 2008, 68(7), S. 2489 ff. beschreiben die Identifikation neuer Methylierungsmarker bei Zervixkarzinom.

Huang et al., Abstract #50, 99th Annual Meeting of the American Association for Cancer Research, San Diego, CA, USA, April 12-16, 2008, ISSN 0197-016X beschreiben die Hypermethylierung von CIDEA und RXFP3 als potentielle epigenetische Marker für Eierstockkrebs.

Der vorliegenden Erfindung liegt somit die Aufgabe zugrunde, ein Verfahren bereitzustellen, mit dem das Zervixkarzinom und vor allem deren Vorstufen frühzeitig und zuverlässig diagnostiziert werden können.

Erfindungsgemäß wird dies durch die Gegenstände in den Patentansprüchen erreicht.

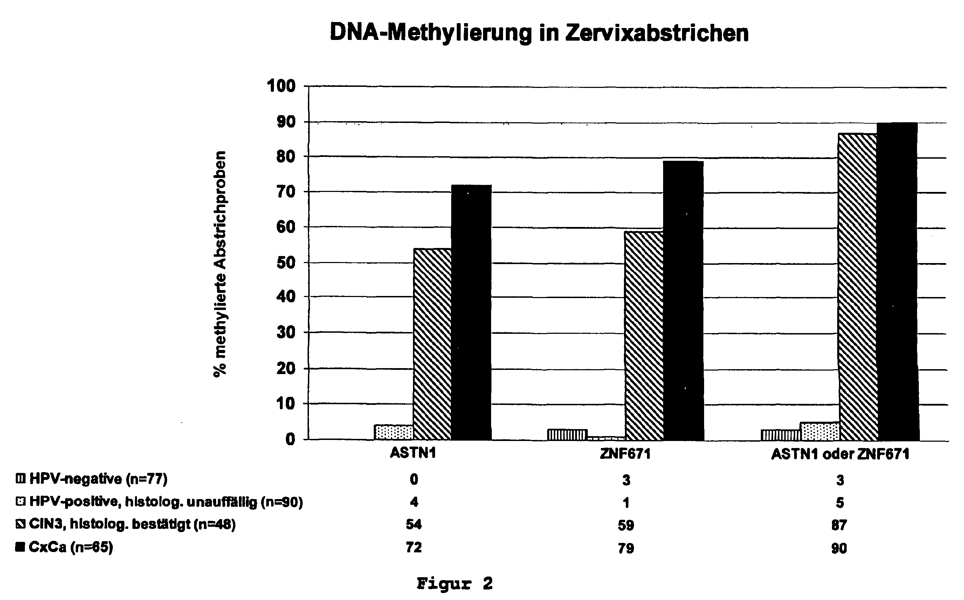

Die vorliegende Erfindung beruht auf den nachfolgend näher beschriebenen Erkenntnissen des Anmelders hinsichtlich des Zusammenhangs zwischen dem Methylierungsstatus bestimmter Gene und der Entwicklung von Karzinomen des Anogenitaltraktes. In Karzinomen und deren Vorstufen werden im Vergleich zum entsprechenden Normalgewebe die Cytosin/Guanin-reichen CpG-Inseln im Upstream-, Promotor- und in promoternahen Exonbereichen von Genen häufig methyliert. Diese Methylierung von Genen geschieht nicht willkürlich, sondern ist von der jeweiligen Tumor-Entität abhängig (Esteller, 2007, Hum. Mol. Genet., 16: R50-R59). Im Rahmen der Erfindung wurden daher Analysen zur Methylierung durchgeführt, mit dem Ziel, Gene zu identifizieren, die besonders häufig in DNA aus Abstrichen von Patientinnen mit histopathologisch bestätigter CIN3 sowie von Patientinnen mit Zervixkarzinom methyliert waren. Des Weiteren sollten die Gene in DNA aus Zervixabstrichen von zytologisch unauffälligen, aber hr-HPV-positiven Frauen nur sehr selten bis gar nicht methyliert sein. Die hier aufgeführte Analyse zeigt erstmals, dass die Methylierung bestimmter Bereiche der Gene ASTN1 und/oder ZNF671 einen wertvollen Marker zur Erkennung von Karzinomen des Anogenitaltraktes (bevorzugt: CIN2+) in einer Probe darstellt (siehe Figur 2). Die vorliegende Erfindung stellt daher verbesserte Methoden zum Nachweis eines Zervixkarzinoms und dessen schwergradige Vorstufen (CIN2+) in einer Probe (z.B. Zellabstrich des Gebärmuttermundes, Zervikalspülung) bereit. Obwohl die Bestimmung des Methylierungsstatus der beiden vorgenannten Gene, wie von den Erfindern gezeigt, schon höchst aussagekräftig ist, kann es zur Absicherung der Diagnose in medizinischen Ausnahmefällen hilfreich sein, auch den Methylierungsstatus eines oder mehrerer weiterer Gene wie DLX1 (distal-less homeobox 1, Transkriptionsfaktor), EDIL3 (EGF-like repeats and discoidin I-like domains 3), ITGA4 (α4-Integrin) und/oder RXFP3 (relaxin/insulin-like family peptide receptor 3) zu ermitteln.

Die vorliegende Erfindung basiert auf dem (direkten oder indirekten) Nachweis des Methylierungsstatus von ASTN1 und ZNF671. Vorzugsweise wird nachgewiesen, ob die Promotorbereiche der Gene ASTN1 und ZNF671 hypermethyliert sind. Da Methylierung meist an Promotorregionen von Genen stattfindet, konzentrieren sich Methoden zum Nachweis der Methylierung der relevanten Gene meist auf diese Regionen. Allerdings können Gene auch in anderen als der Promotorregion methyliert sein, da die GC-reichen CpG-Inseln sich nicht nur dort befinden. Der Nachweis einer Methylierung in solchen weiteren Bereichen kann auch von diagnostischem Nutzen sein und ist damit auch Gegenstand der vorliegenden Erfindung.

In der vorliegenden Erfindung wird der Methylierungsstatus der Gene ASTN1 und ZNF671 vorzugsweise in den Promotorregionen nachgewiesen. Dabei wird untersucht, ob bestimmte Cytosine zu 5-Methylcytosin modifiziert, also methyliert sind oder nicht. In DNA aus einer Probe von einer Frau mit einer hr-HPV Infektion ohne nachweisbare klinische Veränderungen ist die Methylierung in den entsprechenden Positionen der DNA selten bis gar nicht nachweisbar. Bei Frauen mit CIN2+ ist die Wahrscheinlichkeit für eine Methylierung an den gewählten Cytosinresten dagegen hoch.

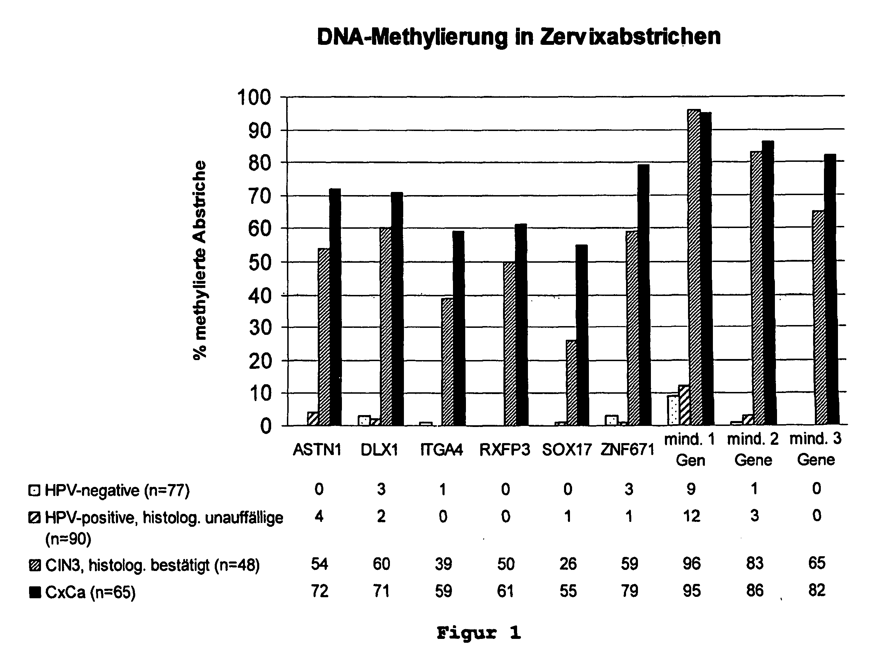

Der spezifische Nachweis der Methylierung von Genen, die während der Karzinogenese im Vergleich zum Normalgewebe auftritt, kann somit den HPV-DNA-Nachweis ergänzen und damit den Pap-Test ersetzen. Die Spezifität des HPV-Tests für den Nachweis von CIN2+ kann durch parallel durchgeführte Methylierungsanalysen erheblich verbessert werden (siehe Figuren 1 und 2). Als Ausgangsmaterial für den Nachweis der DNA-Methylierung dient das gleiche Abstrichmaterial aus den zu testenden Personen wie bisher für den Pap-Test und den HPV-Nachweis.

### Kurze Beschreibung der Figuren

Figur 1: DNA-Methylierung in verschiedenen Abstrichen Graphische Darstellung der Methylierungshäufigkeit der Gene ASTN1, DLX1, EDIL3, ITGA4, RXFP3 und ZNF671 sowie die Häufigkeit der Methylierung jeweils wenigstens eines der Gene in DNA aus Abstrichen von HPV-negativen, unauffälligen (n=77), HPV-positiven, histopathologisch unauffälligen Frauen (n=90), Patientinnen mit histopathologisch bestätigter CIN3 (n=48) und Patientinnen mit Zervixkarzinom (n=65).
Figur 2: DNA-Methylierung in verschiedenen Abstrichen Graphische Darstellung der Methylierungshäufigkeit der Gene ASTN1 und ZNF671 sowie die Häufigkeit der Methylierung jeweils wenigstens eines der Gene in DNA aus Abstrichen von HPV-negativen unauffälligen Frauen (n=77), HPV-positiven histopathologisch unauffälligen Frauen (n=90), Patientinnen mit histopathologisch bestätigter CIN3 (n=48) und Patientinnen mit Zervixkarzinom (n=65).

Somit betrifft die vorliegende Erfindung ein Verfahren zum Nachweis von Gebärmutterhalskrebs oder Vorstufen davon in einer Probe, umfassend die Bestimmung des Methylierungsstatus von ASTN1 (Astrotactin 1) und ZNF671 (Zinkfinger-Protein 671), wobei eine nachweisbare Methylierung eines oder beider Gene ein Hinweis auf Anogenitalkrebs oder einer Vorstufe davon ist.

Optional kann bei uneindeutigen Befunden zusätzlich noch der Methylierungsstatus von einem oder mehreren der Gene DLX1 (distal-less homeobox 1, Transkriptionsfaktor), EDIL3 (EGF-like repeats and discoidin I-like domains 3), ITGA4 (α4-Integrin) oder RXFP3 (relaxin/insulin-like family peptide receptor 3) bestimmt werden.

ASTN1 (Astrotactin 1; accession number NM_004319.1, NM_207108 enthalten in NC_000001.9) ist ein Adhäsionsprotein, das eine wichtige Rolle bei der Migration von neuronalen Zellen spielt. ZNF671 (accession number NM_024883, enthalten in NC_000019.9) ist ein Transkriptionsfaktor mit einem typischen Zinkfinger-Motiv. DLX1 (distal-less homeobox1; accession numbers NM_178120, NM_001038493, enthalten in NC_000002.11) ist ein Transkriptionsfaktor und beeinflusst Zelldifferenzierung. EDIL3 (EGF-like repeats and discoidin I-like domains 3; accession number NM_005711, enthalten in NC_000005.8) ist ein Integrin-Ligand und spielt eine wesentliche Rolle bei der Angiogenese. Er könnte wichtig sein bei der Gefäßwandumstrukturierung und -entwicklung. Zudem fördert EDIL3 die Adhäsion von Endothelzellen. ITGA4 (α-4-Integrin; accession number NM_000885, enthalten in NC_000002.10) gehört zur Familie der α-Integrine, welche zusammen mit jeweils einem β-Integrin als integrale Membranproteine auftreten. Sie dienen als Rezeptoren für Fibronectin und spielen somit eine wesentliche Rolle bei der Zelladhäsion. RXFP3 (relaxin/insulin-like family peptide receptor 3; accession number NM_016568, enthalten in NC_000005.8) dient als G-Protein-gekoppelter Rezeptor für Relaxin 3.

Der hier verwendete Ausdruck "Anogenitalkrebs" umfasst alle Krebsarten des Anogenitaltrakts, also im genitalen, perinealen, analen, und perianalen Bereich, im speziellen ist dies Zervixkarzinom.

Der hier verwendete Ausdruck "Vorstufen" bezieht sich auf schwergradige Vorstufen und umfasst CIN2 und CIN3.

Der hier verwendete Ausdruck "Probe" umfasst jegliche Körperproben, in denen DNA-Methylierung nachgewiesen werden kann. Beispiele solcher Körperproben sind Blut, Abstriche, Sputum, Urin, Stuhl, Liquor, Galle, gastrointestinale Sekrete, Lymphflüssigkeit, Knochenmark, Organpunktate und Biopsien. Insbesondere sind Abstriche und Biopsien angesagt, wenn es sich um den Nachweis von Anogenital-Karzinomen, z. B. Zervixkarzinom, handelt. Der Fachmann kennt geeignete Verfahren und Hilfsmittel zur Probeentnahme. Der Fachmann kennt auch Verfahren und Reagenzien zur DNA-Isolierung aus der Probe, z.B. Extraktion mit Phenol/Chloroform oder mittels kommerzieller Kits.

Der hier verwendete Ausdruck "Methylierungsstatus" bezieht sich auf die Hypermethylierung der genomischen DNA im Bereich der Primer-Bindungsstellen der betreffenden Gene, vorzugsweise in GC-reichen CpG-Inseln im 5'- und Promotorbereich.

Das erfindungsgemäße Diagnoseverfahren wird zur Diagnose von Gebärmutterhalskrebs (Zervixkarzinom) und dessen Vorstufen (zusammen CIN2+) verwendet.

Im erfindungsgemäßen Verfahren wird die Methylierung der Gene ASTN1 und ZNF671 bestimmt. Der hier verwendete Begriff "Methylierung" ist synonym mit dem in der Molekularbiologie gängigen Begriff "Hypermethylierung". Er bezeichnet die vom Normalzustand abweichende Methylierung innerhalb eines allgemein an Guanin und Cytosin und besonders an CG-Dinukleotiden reichen DNA-Abschnittes, einer sog. CpG-Insel. Vorzugsweise handelt es sich bei der für das erfindungsgemäße Verfahren verwendeten Probe um einen Gebärmutterhalsabstrich, der sehr zuverlässige Ergebnisse liefert.

In einer weiteren bevorzugten Ausführungsform des erfindungsgemäßen Verfahrens wird der Methylierungsstatus des Promotorbereichs sowie des 5'- und der promotornahen Exonbereiche eines Gens bestimmt. Diese Bereiche sind besonders aufschlussreich.

Vorzugsweise wird die Methylierung von DNA nach einer vorangegangenen Modifikation von nicht-methylierten Cytosinen durch die Bisulfit-Methode mittels einer sog. methylierungsspezifischen PCR (MSP) unter Verwendung geeigneter Primerpaare nachgewiesen. Bei der Bisulfit-Methode werden unmethylierte Cytosine in Uracil umgewandelt, methylierte Cytosine (5-Methylcytosin) sind vor dieser Umwandlung geschützt. Uracil weist andere Paarungseigenschaften als Cytosin auf, d.h., es verhält sich wie Thymin. Die MSP ist eine etablierte Technik zum Nachweis von DNA-Methylierung. Die MSP beruht auf Primern und evtl. Sonden, die eine Unterscheidung zwischen methylierter und unmethylierter DNA erlauben, d.h., die Ausbildung eines Amplifikationsproduktes zeigt eine Methylierung an, entspricht somit einem positiven Befund.

Das Design der Primer für die Detektion des Methylierungsstatus hängt von der Lokalisierung und Sequenz der Methylierungsbereiche von ASTN1 und ZNF671 sowie von den Methylierungsbereichen von DLX1, EDIL3, ITGA4 und/oder RXFP3 ab, falls optional auch der Methylierungsstatus dieser Gene bestimmt werden soll.

Die in dieser Erfindung verwendbaren methylierungsspezifischen Primer für ASTN1 und ZNF671 binden während des Testverfahrens nur dann an die Bisulfit-modifizierte Proben-DNA, wenn diese an bestimmten Cytosinen innerhalb der Primerbindestellen methyliert war. Waren diese Bereiche vor Bisulfit-Behandlung nicht methyliert, binden auch die Primer nicht, und es entsteht kein Reaktionsprodukt. So weist die Anwesenheit eines Reaktionsproduktes auf eine Methylierung des DNA-Bereiches des jeweiligen Gens und damit auf das mögliche Vorliegen von z.B. im Falle von DNA aus Zervixabstrichen CIN2+ hin.

Besonders bevorzugt ist ein Real-time PCR Verfahren (QMSP), das nicht nur eine qualitative Erfassung der Methylierung, sondern auch eine Quantifizierung der methylierten DNA-Abschnitte erlaubt. Diese MSP kann in einem fluoreszenzbasierten Realtime-Verfahren durchgeführt werden, bei der die Entstehung des methylierungsspezifischen Produktes durch den Einbau eines fluoreszenten Farbstoffes, z. B. SYBR-Green, verfolgt wird. Mit beiden Verfahren (MSP und QMSP) kann die Methylierung von Markergenen in einem hohen Hintergrund von nicht-methylierter DNA nachgewiesen werden (Shames et al., 2007, Cancer Lett. 251:187-98). PCR-basierte Methoden können auch in Hochdurchsatz-Verfahren angewendet werden und sind daher auch für die Krebsvorsorge besonders geeignet.

Noch mehr bevorzugt ist eine auf der "MethyLight"-Technik basierende QMSP, wobei fluoreszierende Sonden für die jeweiligen auf Methylierung zu untersuchenden Genabschnitte verwendet werden. Die Sonde trägt den Fluoreszenzfarbstoff am 5'-Ende und einen Quencher am 3'-Ende. Sie bindet an das PCR-Reaktionsprodukt zwischen den beiden spezifischen Primern. Fluoreszenter Farbstoff wird freigesetzt, sobald die Sonde nach Bindung an die Zielsequenz durch die 5'-3'-Exonucleaseaktivität der DNA-Polymerase abgebaut wird. Die gemessene Fluoreszenz spiegelt die Menge an gebildetem Produkt wider. Durch den Einsatz mehrerer Oligonukleotide und Sonden in einer sog. Multiplex-Reaktion kann die Anzahl der durchzuführenden Reaktionen für zu untersuchende Proben in diesem Verfahren entsprechend vermindert werden (Shames et al., 2007, Cancer Lett. 251:187-98). Geeignete fluoreszente Farbstoffe und Quencher sind dem Fachmann bekannt, z.B. als Fluorophor FAM, HEX^{™}, NED^{™}, ROX^{™}, Texas Red^{™} usw. und als Quencher TAMRA oder BHQ.

In einer besonders bevorzugten Ausführungsform wird das Verfahren der vorliegenden Erfindung als Multiplex-Experiment durchgeführt. Ein solches Multiplex-Experiment erlaubt den Nachweis von, z.B., CIN2+ durch die Analyse des Methylierungsstatus mehrerer Gene, der bekanntermaßen mit dem Vorliegen von CIN2+ in Zusammenhang steht, in einem einzelnen Ansatz pro Probe. Das Multiplexverfahren bietet einige Vorteile, da der Methylierungsstatus des zu testenden Gensets in einer oder zwei Reaktionen pro Probe ermittelt werden kann. Dies führt zu einer erheblichen Ersparnis von Zeit, Probenmaterial und Materialkosten. Im Multiplex-Experiment werden die methylierungsspezifischen Primer für bis zu fünf zu testende Gene eingesetzt. Zusätzlich wird für jedes Gen jeweils ein weiteres spezifisches Olgonukleotid, eine sog. Sonde eingesetzt. Diese Sonde trägt an einem Ende einen fluoreszenten Farbstoff und ist so modifiziert, dass die Fluoreszenz erst dann auftritt, wenn die Sonde spezifisch an während des Experiments entstehende Reaktionsprodukte bindet. Die Sonden tragen unterschiedliche Fluoreszenzgruppen, so dass alle gleichzeitig detektiert werden können. Das erfindungsgemäße Verfahren kann auch mittels der "Microarray"-Technologie durchgeführt werden.

Dem Fachmann sind weitere Methoden zur Bestimmung des Methylierungsstatus bekannt, z.B. solche, die auf einer direkten Bestimmung der Menge des spezifischen Produktes durch Fluoreszenz basieren. So kann für das erfindungsgemäße Diagnoseverfahren auch die "molecular beacon"-Technologie verwendet werden. Molecular Beacons sind Oligonukleotide, die sowohl mit einem Reporter-Fluorophor als auch mit einem Quencher gekoppelt sind. Die Nukleotide am 5'-Ende der Sonde sind zu denen am 3'-Ende komplementär, so dass sich eine für Molecular Beacons charakteristische Sekundärstruktur ausbilden kann. In diesem als Haarnadelstruktur bezeichneten Zustand zeigt der Reporter durch seinen geringen Abstand zum Quencher keine Fluoreszenz. Durch Anlagerung der Schleifen-Region an die komplementäre DNA-Sequenz zwischen den Primern während eines PCR-Zyklus wird der Abstand zwischen Quencher und Reporter vergrößert. Die Reporter-Fluoreszenz kann somit beobachtet werden.

Eine weitere zur Durchführung des erfindungsgemäßen Verfahrens geeignete Methode ist die "Scorpion"-Technologie. Scorpion-Sonden sind komplexe Oligonucleotide, welche die Eigenschaften von Real-Time-PCR-Sonden und PCR-Primern in einem (Uni-Scorpion) oder zwei Molekülen (Bi-Scorpion) vereinigen. Ähnlich den "Molecular Beacons" besitzen sie eine charakteristische Sekundärstruktur mit einer selbstkomplementären Schaft-Region, deren Enden mit einem Reporter-Fluorophor und einem Quencher modifiziert wurden. Zusätzlich tragen diese Sonden am 3'-Ende einen PCR-Primer. Während eines PCR-Zyklus kann mit steigender DNA-Konzentration eine Reporter-Fluoreszenz durch Anlagerung der Loop-Region an eine komplementäre DNA-Sequenz und damit vergrößerten Abstand zwischen Quencher und Reporter beobachtet werden. Zum Nachweis des Bindens der unterschiedlichen Sonden werden diese mit fluoreszierenden Farbstoffmolekülen gekoppelt.

Weiterhin können bei der Durchführung des erfindungsgemäßen Verfahrens positive und/oder negative Kontrollgene, z.B., ein unmethyliertes Kontrollgen co-amplifziert werden.

Es ist bekannt, dass die Methylierung von Genen oft mit einer Transkriptionsblockade und dadurch einer fehlenden Translation in Zusammenhang steht. Somit umfasst das erfindungsgemäße Verfahren auch die indirekte Bestimmung des Methylierungsstatus durch Bestimmung der Konzentration der entsprechenden RNA bzw. des Proteins. Deren Nachweis kann durch übliche Verfahren erfolgen, z.B. (für RNA) über Northern-Blot-Analyse, RT-PCR etc. und (für Proteine) Antikörper-basierte Verfahren oder Verfahren, die auf der Bestimmung einer biologischen Aktivität des Proteins basieren.

Das erfindungsgemäße Verfahren kann beispielsweise auf den folgenden Schritten beruhen:
(a) Vom Zellabstrich der zu testenden Person wird DNA nach einem Standard-Verfahren isoliert, z. B. QiaAmp DNA-Mini Kit, nach Vorschrift des Herstellers (QIAGEN, Hilden, Deutschland);
(b) Vorzugsweise wird in einem zweiten Schritt untersucht, ob die zu analysierende Probe hr-HPV-DNA enthält. Dieser Nachweis erfolgt mit einem bereits etablierten Verfahren wie z.B. dem GP5+/6+-PCR EIA-Verfahren (Jacobs et al., 1995, J Clin Microbiol 33:901-905). Sämtliche hr-HPV Typen (HPV16, 18, 26, 31, 33, 35, 39, 45, 51, 52, 53, 56, 58, 59, 66, 68, 73, 82) sollten erfasst werden. Durch den Nachweis eines house-keeping Gens, z.B. ß-Globin oder ß-Actin, wird sichergestellt, dass in der Probe eine ausreichende Menge an DNA von ausreichender Qualität vorliegt, die auch amplifizierbar ist;
(c) Bei einer HPV-negativen Probe ist das Vorliegen einer CIN2+ nahezu 100% ausgeschlossen (Cuzick et al., 2006; Int. J. Cancer. 119:1095-1101). Diese Proben müssen daher nicht weiter untersucht werden. Die Differenzierung zwischen Frauen mit einem hr-HPV-positiven Befund entweder mit oder ohne CIN2+ erfolgt dann über die Bestimmung des Methylierungsstatus der Gene ASTN1 und ZNF671;
(d) Dazu wird die DNA der hr-HPV positiven Proben nach der Bisulfit-Methode, z. B. mittels eines kommerziellen Kits (z. B. Methylation Gold Kit, Zymo Research, Orange, CA, USA), chemisch konvertiert. Dabei werden durch Behandlung mit Natrium-Bisulfit und nachfolgende alkalische Hydrolyse alle nicht methylierten Cytosine der DNA-Probe in Uracile umgewandelt;
(e) Die relevante DNA wird mittels spezifischer Primer für die methylierte Form der jeweiligen Genomabschnitte durch PCR amplifiziert und analysiert;
(f) Zum Nachweis der Methylierung der Gene ASTN1 und ZNF671 in einer methylierungsspezifischen PCR oder QMSP können die folgenden methylierungsspezifischen Primer eingesetzt werden:

| | |
|---|---|
| ASTN1_F | CGTAAGCGTTGTTAGCGTAGC |
| ASTN1_R | CGCGAAATCGAAACGAAAACG |
| ZNF671_F | CGGAGGACGTAGTATTTATTCGC |
| ZNF671_R | CTACGTCCCCGATCGAAACG |

Das erfindungsgemäße Diagnoseverfahren beschränkt sich aber nicht auf den Einsatz dieser Primer für der Nachweis der Methylierung der Gene ASTN1 und ZNF671. Sie umfasst auch Primer anderer Sequenzen, die zum Nachweis der Methylierung der beiden Gene dienen können.
(g) Zum Nachweis der Methylierung der Gene ASTN1 und ZNF671 durch MethyLight-Analyse werden zusätzlich zu den vorstehend beschriebenen Primern fluoreszierende Sonden eingesetzt:

| | |
|---|---|
| ASTN1 | GTAATTCGTTTGTTTCGTAAGTTGTTCG |
| ZNF671 | CGTGGGCGCGGACAGTTGTCGGGAGCG |

Die Sonden werden, je nach Applikation, mit entsprechenden fluoreszenten Farbstoffen gekoppelt. Die Erfindung beschränkt sich aber nicht auf den Einsatz dieser Sonden für den Nachweis der Methylierung der Gene ASTN1 und ZNF671. Sie umfasst auch Sonden mit anderen Sequenzen, die zum Nachweis der Methylierung der beiden Gene dienen können.

Eine Quantifizierung der Methylierung der Markergene ist nicht zwingend. Entscheidend ist, dass, z.B., durch MSP oder QMSP mindestens eine Zelle mit methylierten Markergen(en) in einem Hintergrund von 1000 Zellen ohne methylierte(s) Markergen(e) nachgewiesen werden kann.

In einer weiteren besonders bevorzugten Ausführungsform des erfindungsgemäßen Verfahrens wird zusätzlich die HPV-DNA in der Probe quantifiziert. Dies ermöglicht eine Abschätzung der Anzahl HPV-positiver Zellen in der durch Bisulfitbehandlung modifizierten DNA durch PCR mit entsprechend an die Bisulfit-Modifikation angepassten hr-HPV-spezifischen Oligonukleotidprimern. Die Primer erlauben vorzugsweise die Amplifikation in der konservierten L1-Region des HPV-Genoms und können beispielsweise die folgenden Sequenzen umfassen:

| | |
|---|---|
| HPV-F | GGTTATAATAATGGTATTTGTTGGG |
| HPV_R | TAAAAAATAAACTATAAATCATATTCC |

In einer weiteren bevorzugten Ausführungsform wird die Methylierung einer oder mehrerer der Gene DLX1, EDIL3, ITGA4 und/oder RXFP3 überprüft. Hierzu verwendbare Primer sind:

| | |
|---|---|
| DLX1_F | TATCGGGATTCGCGTTTGTAC |
| DLX1_R | CGACCGAACTAAAACTCAACTCG |
| EDIL3_F | GTTTTCGGCGGTTCGTTC |
| EDIL3_R | CGAACGCTCGACTATCGC |
| ITGA4_F | CGAATTCGGTTTTCGAAGGGTC |
| ITGA4_R | CACGACCGAATAACCGAACAAC |
| RXFP3_F | ATTTCGGAAAGCGTTTTTCGC |
| RXFP3_R | CTACGTCTCTCCGCGATTATC |

Zum Nachweis der Methylierung der Gene DLX1, EDIL3, ITGA4 und RXFP3 durch MethyLight-Analyse werden zusätzlich zu den vorstehend beschriebenen Primern fluoreszierende Sonden eingesetzt:

| | |
|---|---|
| DLX1 | CGTAAACGTTAGCTGTTCTGGAAACCG |
| EDIL3 | TCGTAGTCGTCGCGCGGAGAATA |
| ITGA4 | TTCGATCGGTCGTTTTTATAACG |
| RXFP3 | GCGTTTTGGGATTACGTATGTTTTTTGG |

Die Sonden werden, je nach Applikation, mit entsprechenden fluoreszenten Farbstoffen gekoppelt.

Ein weiterer Gegenstand der vorliegenden Erfindung ist ein Kit zur Durchführung eines erfindungsgemäßen Verfahrens. Ein solches Kit umfasst genspezifische Primer oder Primerpaare zur Bestimmung des Methylierungsstatus von ASTN1 (Astrotactin 1) und ZNF671 (Zinkfingerprotein, Transkriptionsfaktor), vorzugsweise die vorstehend näher definierten Primer bzw. Primerpaare. In einer weiteren Ausführungsform kann der Kit zusätzlich genspezifische Primer oder Primerpaare zur Bestimmung des Methylierungsstatus von DLX1 (distal-less homeobox 1) EDIL3 (EGF-like repeats and discoidin I-like domains 3), ITGA4 (α4-Integrin) und/oder RXFP3 (relaxin/insulin-like family peptide receptor 3), vorzugsweise die vorstehend näher definierten Primer bzw. Primerpaare, enthalten.

Das Kit kann zusätzlich auch eine Sonde wie vorstehend näher beschrieben und/oder ein Primerpaar für den Nachweis einer HPV-Infektion enthalten.

Schließlich kann das Kit in getrennten Behältern zusätzlich mindestens einen der folgenden Bestandteile enthalten:
(a) Reagerizien zur Isolierung von DNA;
(b) Enzyme zur DNA-Amplifikation;
(c) Natriumbisulfit;
(d) einen oder mehrere Puffer.

Von den einzelnen Komponenten des Kits können ein oder mehrere Vertreter vorliegen.

Mit der vorliegenden Erfindung ist es somit möglich, Zervixkarzinome frühzeitig zu diagnostizieren. Insbesondere können Vorstufen dieser Karzinome frühzeitig erkannt werden. Zu betonen ist, dass zwischen hr-HPV positiven Frauen mit oder ohne klinisch relevante Gewebeveränderung, z.B. CIN2+, unterschieden werden kann. Kennzeichnend ist ferner, dass die durch ein erfindungsgemäßes Verfahren erzielten Ergebnisse nicht einer subjektiven Bewertung unterliegen, wodurch z.B. die falschnegativen bzw. falsch-positiven Ergebnisse eines Pap-Tests vermieden werden können. Des Weiteren zeichnet sich das vorliegende erfindungsgemäße Diagnoseverfahren durch schnelle und einfache Handhabung aus, wodurch es für große Screening-Maßnahmen, insbesondere auch in Ländern der Dritten Welt, geeignet ist. Somit liefert die vorliegende Erfindung einen wichtigen Beitrag zur modernen Diagnostik des Zervixkarzinoms oder Vorstufen davon.

In dieser Hinsicht muss hervorgehoben werden, dass durch die erfindungsgemäße Kombination der Bestimmung des Methylierungsstatus' von ASTN1 und ZNF671 eine überragende diagnostische Sicherheit der Erkennung von CIN2+ Läsionen erreicht werden kann, die über die Einzelbestimznung anderer Gene hinausgeht. So ist beispielsweise RXFP3 (Huang et al.) ein mehr als 10% schlechterer Marker als ASTN1 und ZNF671 (s. Fig. 1). Die oben erwähnte Bestimmung des Methylierungsstatus der weiteren Markergene ist daher nur in Ausnahmefällen noch zusätzlich notwendig und wird erfindungsgemäß nur zur Diagnoseabsicherung unternommen.

Die Erfindung wird durch das folgende Beispiel erläutert.

### Beispiel 1

### DNA-Methylierung in Abstrichen

Untersucht wurden (1) Abstriche von 77 HPV-negativen Frauen,(2) Abstriche von 90 hr-HPV positiven Frauen, bei denen es keinen Anhalt (histopathologisch) für eine Gewebeveränderung gab; (3) Abstriche von 48 hr-HPV positiven Frauen mit einer histopathologisch gesicherten CIN3 und (4) Abstriche von 65 hr-HPV positiven Frauen mit einem histopathologisch gesicherten Zervixkarzinom.

Die Ergebnisse sind in Figur 2 dargestellt. Fig. 2 zeigt die Methylierungshäufigkeit der Gene ASTN1 und ZNF671 in den einzelnen Gruppen. Eine Methylierung wenigstens eines der Gene trat in 87% der CIN3- und 90% der Zervixkarzinomproben auf, hingegen nur in 3% der hr-HPV-negativen und 5% der hr-HPV positiven Frauen ohne Gewebeveränderungen. Durch die Kombination der beiden Gene kann somit eine nahezu 100%ige Diagnosesicherheit erreicht werden. Dies zeigt die Zuverlässigkeit des erfindungsgemäßen Verfahrens.

Die PCR für den Nachweis sowohl einer HPV-Infektion als auch für den Nachweis der methylierten Gene ist sehr empfindlich. Das vorliegende Beispiel beruht auf der jeweiligen qualitativen Bestimmung des HPV-Status und des Methylierungsstatus der Markergene. Durch eine quantitative Bestimmung der HPV DNA kann ein Grenzwert definiert werden, der einer Mindestanzahl HPV-positiver Zellen in der Probe entspricht. Dadurch kann die Spezifität der methylierungsspezifischen PCR für den Nachweis von CIN2+ weiter erhöht werden.

### Literatur

Cuzick et al., 2006; Int J Cancer 119:1095-1101
Esteller, 2007, Hum. Mol. Genet. 16: R50-R59
Jacobs et al., 1995, J Clin Mircobiol 33:901-905
Shames et al., 2007, Cancer Lett. 251:187-98
Yu et al., 2007, Clin Cancer Res 13: 7296 - 7304
Wentzensen et al., 2009; Gynecologic Oncology 112, 293-299
Huang et al., Abstract #50, 99th AACR Annual Meeting, April 12-16, 2008, San Diego

## Patentansprüche

1. Verfahren zum Nachweis von Anogenitalkrebs oder Vorstufen davon in einer Probe, umfassend die Bestimmung des Methylierungsstatus von ASTN1 (Astrotactin 1) und ZNF671 (Zinkfingerprotein, Transkriptionsfaktor), wobei ASTN1 und/oder ZNF671 in einer positiven Probe methyliert sind, und wobei der Anogenitalkrebs Gebärmutterhalskrebs ist.

2. Verfahren nach Anspruch 1, wobei die Probe ein Gebärmutterhalsabstrich ist.

3. Verfahren nach Anspruch 1 oder 2, wobei der Methylierungsstatus des Promotorbereichs bestimmt wird.

4. Verfahren nach einem der Ansprüche 1 bis 3, wobei der Methylierungsstatus der Gene mittels methylierungsspezifischer PCR (MSP) bestimmt wird, wobei bevorzugt die MSP eine quantitative MSP (QMSP) ist.

5. Verfahren nach Anspruch 4, wobei die quantitative MSP auf der MethyLight-Technik basiert.

6. Verfahren nach einem der Ansprüche 1 bis 5, wobei das Verfahren ein Multiplex-Verfahren ist.

7. Verfahren nach Anspruch 4, 5 oder 6, wobei Primerpaare verwendet werden, die die folgende Sequenz umfassen:
(a) CGTAAGCGTTGTTAGCGTAGC (ASTN1_F) und CGCGAAATCGAAACGAAAACG (ASTN1_R);
(b) CGGAGGACGTAGTATTTATTCGC (ZNF671_F) und CTACGTCCCCGATCGAAACG (ZNF671_R).

8. Verfahren nach Anspruch 5 oder 6, wobei ein Primerpaar oder mehrere Primerpaare wie in Anspruch 7 definiert verwendet werden und zusätzlich mindestens eine Sonde, die eine der folgenden Sequenzen umfasst:
(a) GTAATTCGTTTGTTTCGTAAGTTGTTCG (ASTN1);
(b) CGTGGGCGCGGACAGTTGTCGGGAGCG (ZNF671).

9. Verfahren nach einem der Ansprüche 1 bis 8, zusätzlich den Nachweis einer HPV-Infektion umfassend, wobei der HPV-Nachweis bevorzugt über PCR erfolgt.

10. Verfahren nach Anspruch 9, wobei bei der PCR ein Primerpaar verwendet wird, das folgende Sequenzen umfasst:
(a) GGTTATAATAATGGTATTTGTTGGG (HPV-F); und
(b) TAAAAAATAAACTATAAATCATATTCC (HPV_R).

11. Kit für die Verwendung zum Nachweis von Anogenitalkrebs oder Vorstufen davon in einer Probe, umfassend:
Primer oder Primerpaare zur Bestimmung des Methylierungsstatus von ASTN1 (Astrotactin 1) und ZNF671 (Zinkfingerprotein, Transkriptionsfaktor), wobei die Primer spezifisch an die methylierte Form von zuvor mit der Bisulfit-Methode behandelten Proben-DNA binden, und wobei der Anogenitalkrebs Gebärmutterhalskrebs ist.

12. Kit nach Anspruch 11, enthaltend die Primer/Primerpaare wie in Anspruch 7 definiert.

13. Kit nach Anspruch 11 oder 12, enthaltend zusätzlich eine Sonde wie in Anspruch 8 definiert.

14. Kit nach einem der Ansprüche 11 bis 13, zusätzlich ein Primerpaar für den Nachweis einer HPV-Infektion enthaltend, wobei das Primerpaar bevorzugt das in Anspruch 10 definierte Primerpaar ist.

15. Kit nach einem der Ansprüche 11 bis 14, der in getrennten Behältern zusätzlich mindestens einen der folgenden Bestandteile enthält:
(a) Reagenzien zur Isolierung von DNA;
(b) Enzyme zur DNA-Amplifikation;
(c) Natriumbisulfit;
(d) einen oder mehrere Puffer.

## Claims

1. A method for detecting anogenital cancer or preliminary stages thereof in a sample, comprising determining the methylation status of ASTN1 (astrotactin 1) and ZNF671 (zinc finger protein, transcription factor), wherein ASTN1 and/or ZNF671 are methylated in a positive sample and wherein the anogenital cancer is cancer of the cervix uteri.

2. The method according to claim 1, wherein the sample is a cervical smear.

3. The method according to claim 1 or 2, wherein the methylation status of the promoter region is determined.

4. The method according to any of claims 1 to 3, wherein the methylation status of the genes is determined by means of methylation-specific PCR (MSP), preferably wherein the MSP is a quantitative MSP (QMSP).

5. The method according to claim 4, wherein the quantitative MSP is based on the MethyLight technique.

6. The method according to any of claims 1 to 5, wherein the method is a multiplex method.

7. The method according to claim 4, 5 or 6, wherein primer pairs are used which comprise the following sequence:
(a) CGTAAGCGTTGTTAGCGTAGC (ASTN1_F) and CGCGAAATCGAAACGAAAACG (ASTN 1_R);
(b) CGGAGGACGTAGTATTTATTCGC (ZNF671_F) and CTACGTCCCCGATCGAAACG (ZNF671_R).

8. The method according to claim 5 or 6, wherein a primer pair or several primer pairs as defined in claim 7 are used and additionally at least one probe which comprises one of the following sequences:
(a) GTAATTCGTTTGTTTCGTAAGTTGTTCG (ASTN1);
(b) CGTGGGCGCGGACAGTTGTCGGGAGCG (ZNF671).

9. The method according to any of claims 1 to 8, additionally comprising the detection of an HPV infection, preferably wherein the HPV detection is done via PCR.

10. The method according to claim 9, wherein in the PCR a primer pair is used which comprises the following sequences:
(a) GGTTATAATAATGGTATTTGTTGGG (HPV_F); and
(b) TAAAAAATAAACTATAAATCATATTCC (HPV_R).

11. Kit for use of detecting anogenital cancer or preliminary stages thereof in a sample, comprising:
primers or primer pairs for determining the methylation status of ASTN1 (astrotactin 1) and ZNF671 (zinc finger protein, transcription factor), wherein the primers specifically bind to the methylated form of sample DNA previously treated with the bisulfite method and wherein the anogenital cancer is cancer of the cervix uteri.

12. Kit according to claim 11, containing the primers/primer pairs as defined in claim 7.

13. Kit according to claim 11 or 12, additionally containing a robe as defined in claim 8.

14. Kit according to any of claims 11 to 13, additionally containing a primer pair for the detection of an HPV infection, preferably wherein the primer pair is the primer pair defined in claim 10.

15. Kit according to any of claims 11 to 14, containing in separate containers additionally at least one of the following constituents:
(a) reagents for isolating DNA;
(b) enzyme for DNA amplification;
(c) sodium bisulfite;
(d) one or more buffers.

## Revendications

1. Procédé de diagnostic du cancer anogénital ou de stades précoces de ce dernier dans un échantillon, comprenant la détermination du statut de méthylation d'ASTN1 (astrotactine 1) et de ZNF671 (protéine de doigt de zinc, facteur de transcription), dans lequel l'ASTN1 et/ou le ZNF671 sont méthylés dans un échantillon positif, et dans lequel le cancer anogénital est le cancer du col de l'utérus.

2. Procédé selon la revendication 1, dans lequel l'échantillon est un frottis du col de l'utérus.

3. Procédé selon la revendication 1 ou 2, dans lequel est déterminé le statut de méthylation de la zone de promoteur.

4. Procédé selon l'une des revendications 1 à 3, dans lequel le statut de méthylation des gènes est déterminé à l'aide de PCR spécifique à la méthylation (MSP), le MSP étant de préférence un MSP quantitatif (QMSP).

5. Procédé selon la revendication 4, dans lequel le MSP quantitatif se base sur la technique méthylight.

6. Procédé selon l'une des revendications 1 à 5, dans lequel le procédé est un procédé multiplex.

7. Procédé selon la revendication 4, 5 ou 6, dans lequel sont utilisées des paires d'amorces qui comportent la séquence suivante:
(a) CGTAAGCCTTAGGTAGC (ASTN1_F) et CGCGAAATCGAAACGAAAACG (ASTN1_R);
(b) CGGAGGACGTATTTATTCGC (ZNF671_F) et CTACGTCCCCGATCGAAACG (ZNF671_R).

8. Procédé selon la revendication 5 ou 6, dans lequel une paire d'amorces ou plusieurs paires d'amorces telle(s) que définie(s) à la revendication 7 sont utilisées et en outre au moins une sonde qui comporte l'une des séquences suivantes:
(a) GTAATTCGTTTGTTTCGTAAGTTGTTCG (ASNT1);
(b) CGTGGGCGCGGACAGTTGTCGGGAGCG (ZNF671).

9. Procédé selon l'une des revendications 1 à 8, comprenant en outre le diagnostic d'une infection par HPV, le diagnostic de HPV ayant lieu de préférence par PCR.

10. Procédé selon la revendication 9, dans lequel est utilisée, lors du PCR, une paire d'amorces qui contient les séquences suivantes:
(a) GGTTATAATAATGGTATTTGTTGGG (HPV-F); et
(b) TAAAAAATAAACTATAAATCATATTCC (HPV_R).

11. Kit destiné à être utilisé pour le diagnostic du cancer anogénital ou de stades précoces de ce dernier dans un échantillon, comprenant:
des amorces ou des paires d'amorces pour la détermination du statut de méthylation d'ASTN1 (astrotactine 1) et de ZNF671 (protéine de doigt de zinc, facteur de transcription), où les amorces lient de manière spécifique à la forme méthylée d'ADN des échantillons traités au préalable par la méthode de bisulfite, et où le cancer anogénital est le cancer du col de l'utérus.

12. Kit selon la revendication 11, contenant les amorces/paires d'amorces telles que définies à la revendication 7.

13. Kit selon la revendication 11 ou 12, contenant en outre une sonde telle que définie à la revendication 8.

14. Kit selon l'une des revendications 11 à 13, contenant en outre une paire d'amorces pour le diagnostic d'une infection par HPV, dans lequel la paire d'amorces est de préférence la paire d'amorces définie à la revendication 10.

15. Kit selon l'une des revendications 11 à 14, qui contient dans des récipients séparés en outre au moins l'un des composants suivants:
(a) réactifs pour l'isolation de l'ADN;
(b) enzymes pour l'amplification de l'ADN;
(c) bisulfite de sodium;
(d) un ou plusieurs tampons.


### SEQUENCE LISTING

<110> universitätsklinikum Jena Körperschaft des öffentlichen Rechts und Teilkörperschaft der Friedrich-Schiller Universität Jena
<120> Verfahren zur frühen Diagnose von Karzinomen des Anogenitaltraktes
<130> D 2538EP
<140> EP09011941
   <141> 2009-09-18
<160> 20
<170> PatentIn version 3.3
<210> 1
   <211> 21
   <212> DNA
   <213> artificial sequence
<220>
   <223> primer ASTN1_F
<400> 1
   cgtaagcgtt gttagcgtag c 21
<210> 2
   <211> 21
   <212> DNA
   <213> artificial sequence
<220>
   <223> primer ASTN1_R
<400> 2
   cgcgaaatcg aaacgaaaac g 21
<210> 3
   <211> 21
   <212> DNA
   <213> artificial sequence
<220>
   <223> primer DLX1_F
<400> 3
   tatcgggatt cgcgtttgta c 21
<210> 4
   <211> 23
   <212> DNA
   <213> artificial sequence
<220>
   <223> primer DLX1_R
<400> 4
   cgaccgaact aaaactcaac tcg 23
<210> 5
   <211> 18
   <212> DNA
   <213> artificial sequence
<220>
   <223> primer EDIL3_F
<400> 5
   gttttcggcg gttcgttc 18
<210> 6
   <211> 18
   <212> DNA
   <213> artificial sequence
<220>
   <223> primer EDIL3_R
<400> 6
   cgaacgctcg actatcgc 18
<210> 7
   <211> 22
   <212> DNA
   <213> artificial sequence
<220>
   <223> primer ITGA4_F
<400> 7
   cgaattcggt tttcgaaggg tc 22
<210> 8
   <211> 22
   <212> DNA
   <213> artificial sequence
<220>
   <223> primer ITGA4_R
<400> 8
   cacgaccgaa taaccgaaca ac 22
<210> 9
   <211> 21
   <212> DNA
   <213> artificial sequence
<220>
   <223> primer RXFP3
<400> 9
   atttcggaaa gcgtttttcg c 21
<210> 10
   <211> 21
   <212> DNA
   <213> artificial sequence
<220>
   <223> primer RXFP3_R
<400> 10
   ctacgtctct ccgcgattat c 21
<210> 11
   <211> 23
   <212> DNA
   <213> artificial sequence
<220>
   <223> primer ZNF671_F
<400> 11
   cggaggacgt agtatttatt cgc 23
<210> 12
   <211> 20
   <212> DNA
   <213> artificial sequence
<220>
   <223> primer ZNF671_R
<400> 12
   ctacgtcccc gatcgaaacg 20
<210> 13
   <211> 28
   <212> DNA
   <213> artificial sequence
<220>
   <223> probe ASTN1
<400> 13
   gtaattcgtt tgtttcgtaa gttgttcg 28
<210> 14
   <211> 27
   <212> DNA
   <213> artificial sequence
<220>
   <223> probe DLX1
<400> 14
   cgtaaacgtt agctgttctg gaaaccg 27
<210> 15
   <211> 23
   <212> DNA
   <213> artificial sequence
<220>
   <223> probe EDIL3
<400> 15
   tcgtagtcgt cgcgcggaga ata 23
<210> 16
   <211> 23
   <212> DNA
   <213> artificial sequence
<220>
   <223> probe ITGA4
<400> 16
   ttcgatcggt cgtttttata acg 23
<210> 17
   <211> 28
   <212> DNA
   <213> artificial sequence
<220>
   <223> probe RXFP3
<400> 17
   gcgttttggg attacgtatg ttttttgg 28
<210> 18
   <211> 27
   <212> DNA
   <213> artificial sequence
<220>
   <223> probe ZNF671
<400> 18
   cgtgggcgcg gacagttgtc gggagcg 27
<210> 19
   <211> 25
   <212> DNA
   <213> artificial sequence
<220>
   <223> primer HPV_F
<400> 19
   ggttataata atggtatttg ttggg 25
<210> 20
   <211> 27
   <212> DNA
   <213> artificial sequence
<220>
   <223> primer HPV_R
<400> 20
   taaaaaataa actataaatc atattcc 27